(19) **Europäisches Patentamt**
**European Patent Office**
**Office européen des brevets**

(11) **EP 1 458 316 B1**

(12) **FASCICULE DE BREVET EUROPEEN**

(45) Date de publication et mention
de la délivrance du brevet:
**14.07.2010  Bulletin 2010/28**

(21) Numéro de dépôt: **02801186.4**

(22) Date de dépôt: **30.12.2002**

(51) Int Cl.:
***A61F 5/00*** *(2006.01)*

(86) Numéro de dépôt international:
**PCT/FR2002/004589**

(87) Numéro de publication internationale:
**WO 2003/055420 (10.07.2003 Gazette 2003/28)**

(54) **BALLON INTRA-GASTRIQUE ET PROCEDE DE FABRICATION CORRESPONDANT**

INTRAGASTRALER BALLON UND VERFAHREN ZUR HERSTELLUNG DES BALLONS

INTRAGASTRIC BALLOON AND METHOD FOR MAKING SAME

(84) Etats contractants désignés:
**AT BE BG CH CY CZ DE DK EE ES FI FR GB GR IE IT LI LU MC NL PT SE SI SK TR**

(30) Priorité: **28.12.2001  FR 0117102**

(43) Date de publication de la demande:
**22.09.2004  Bulletin 2004/39**

(73) Titulaire: **Compagnie Europeenne d'Etude et de Recherche de Dispositifs pour l'Implantation par Laparoscopie 38205 Vienne Cedex (FR)**

(72) Inventeurs:
• **LOINTIER, Patrice, Henri F-63400 Chamalières (FR)**

• **VERDIER, Alain, Jean, Charles F-63400 Chamalières (FR)**
• **BORY, Roger-Michel F-01700 Miribel (FR)**

(74) Mandataire: **Croonenbroek, Thomas Jakob et al Cabinet Innovincia 11, Avenue des Tilleuls 74200 Thonon-les-Bains (FR)**

(56) Documents cités:
**US-A- 4 607 618    US-A- 4 694 827
US-A- 4 696 288    US-A- 4 739 758
US-A- 5 084 061    US-A- 5 234 454**

**Description**

DOMAINE TECHNIQUE

La présente invention se rapporte au domaine technique des dispositifs artificiels destinés à assurer le traitement de l'obésité, en particulier de l'obésité morbide, et tout particulièrement ceux consistant à réduire, de manière artificielle, le volume de la cavité gastrique, en vue de créer rapidement une sensation de satiété chez le patient.

L'invention concerne un ballon intra-gastrique pour le traitement de l'obésité, destiné à être implanté dans l'estomac d'un patient pour réduire le volume de l'estomac, ledit ballon comprenant une enveloppe souple délimitant un volume interne prédéterminé, ladite enveloppe souple étant réalisée en un matériau élastomère.

L'invention concerne également un procédé de fabrication d'un ballon intra-gastrique pour le traitement de l'obésité, ledit ballon étant destiné à être implanté dans l'estomac d'un patient pour réduire le volume de l'estomac.

Le document US-A-5084061 représente l'état de la technique le plus proche et détermine le préambule de la revendication 1.

TECHNIQUE ANTERIEURE

Pour traiter les patients atteints d'obésité, notamment ceux présentant un rapport poids / taille ne nécessitant pas le recours à des dispositifs et méthodes chirurgicales invasives, lourdes et traumatisantes, tels que l'implantation par voie chirurgicale d'anneau gastrique ou également pour traiter les patients dont la surcharge pondérale trop importante est considérée comme un risque vis-à-vis d'une intervention chirurgicale, il est connu d'implanter directement dans l'estomac du patient un corps étranger, de volume suffisant pour réduire l'espace disponible pour les aliments, tout en réduisant leur vitesse de passage.

Ces corps étrangers sont implantés par voie orale, et se présentent généralement sous la forme de ballons dits intra-gastriques, formés par une poche souple réalisée en un matériau élastomère biocompatible qui est implantée directement dans l'estomac du patient.

Le ballon présente un orifice dans lequel est installée une valve, ces deux éléments formant un moyen de connexion dans lequel le chirurgien, avant avoir implanté dans sa forme non expansée le ballon, insère un organe de connexion, en général un cathéter relié à une source de fluide (liquide physiologique), de manière à pouvoir procéder au gonflage ou à l'expansion du ballon dans l'estomac.

De tels ballons intra-gastriques sont largement connus et, s'ils fournissent des résultats intéressants en matière de perte de poids, puisqu'ils réduisent la cinétique de passage des aliments et contribuent effectivement à générer rapidement une sensation de satiété, ils souffrent néanmoins d'inconvénients non négligeables.

En particulier, ils s'avèrent être souvent difficilement supportés par les patients en raison du poids important du ballon, qui renferme un volume de liquide conséquent, de l'ordre de 600 mL.

Par ailleurs, leur mise en place peut parfois s'avérer délicate, de même que leur technique d'expansion et de manipulation.

Enfin, il s'avère que la forme extérieure des ballons intra-gastriques connus à ce jour n'est pas à même de bloquer de manière suffisante, et pendant une durée conséquente, le passage des aliments dans le reste du tractus digestif, alors que l'on cherche précisément à prolonger autant que possible la sensation de satiété.

EXPOSE DE L'INVENTION

Les objets assignés à l'invention visent en conséquence à porter remède aux différents inconvénients énumérés précédemment et à proposer un nouveau ballon intra-gastrique expansible pour le traitement de l'obésité, destiné à être implanté dans l'estomac d'un patient et qui, tout en étant d'un volume suffisant, soit particulièrement léger et bien supporté par le patient.

Un autre objet de l'invention vise à proposer un nouveau ballon intra-gastrique qui soit particulièrement bien équilibré lors de son expansion radiale, et dont l'implantation est facilitée.

Un autre objet de l'invention vise à proposer un nouveau ballon intra-gastrique dont l'implantation, et en particulier l'expansion, est particulièrement simplifiée et rapide.

Un autre objet de l'invention vise à proposer un nouveau ballon intra-gastrique particulièrement résistant dont les pertes de fluide sont réduites.

Un autre objet de l'invention vise à proposer un nouveau ballon de conception simplifiée et présentant de bonnes résistances, notamment mécaniques, en général.

Un autre objet de l'invention vise à proposer un nouveau ballon intra-gastrique permettant d'augmenter sensiblement le blocage des aliments dans l'estomac.

Un autre objet de l'invention vise également à proposer un nouveau dispositif chirurgical pour le traitement de l'obésité, qui permet une expansion particulièrement simplifiée et rapide du ballon vers son volume prédéterminé de fonctionnement.

Un autre objet de l'invention vise à proposer un nouveau procédé de fabrication d'un ballon intra-gastrique qui est particulièrement simple et efficace à mettre en oeuvre, tout en permettant d'obtenir un ballon présentant une excellente étanchéité.

Les objets assignés à l'invention sont atteints à l'aide d'un ballon intra-gastrique expansible pour le traitement de l'obésité comme défini par la revendication 1.

Les objets assignés à l'invention sont également atteints à l'aide d'un procédé de fabrication d'un ballon intra-gastrique comme défini par la revendication 5.

DESCRIPTIF SOMMAIRE DES DESSINS

D'autres objets et avantages de l'invention apparaîtront mieux à la lecture de la description qui suit, ainsi qu'à l'aide des dessins annexés, donnés à titre purement illustratif et non limitatif, dans lesquels :

- La figure 1 illustre, selon une vue en perspective, un ballon intra-gastrique conforme à l'invention dans sa position d'expansion maximale, et équipé d'un organe de connexion tubulaire.

- La figure 2 illustre, selon une vue en coupe transversale longitudinale identique à celle de la figure 1, un ballon intra-gastrique.

- La figure 3 illustre, selon une vue en coupe transversale longitudinale partielle, un détail de réalisation et de montage d'un organe de connexion tubulaire, inséré dans un ballon intra-gastrique.

- Les figures 4, 5 et 6 illustrent, selon des vues schématiques en coupe transversale, les étapes principales du procédé de fabrication d'un ballon intra-gastrique bi-poches.

- Les figures 7 à 10 illustrent des variantes de ballons intra-gastriques.

- Les figures 11 et 12 illustrent des moyens permettant la fabrication de ballons intra-gastriques conformes à un objet de l'invention.

MEILLEURE MANIERE DE REALISER L'INVENTION

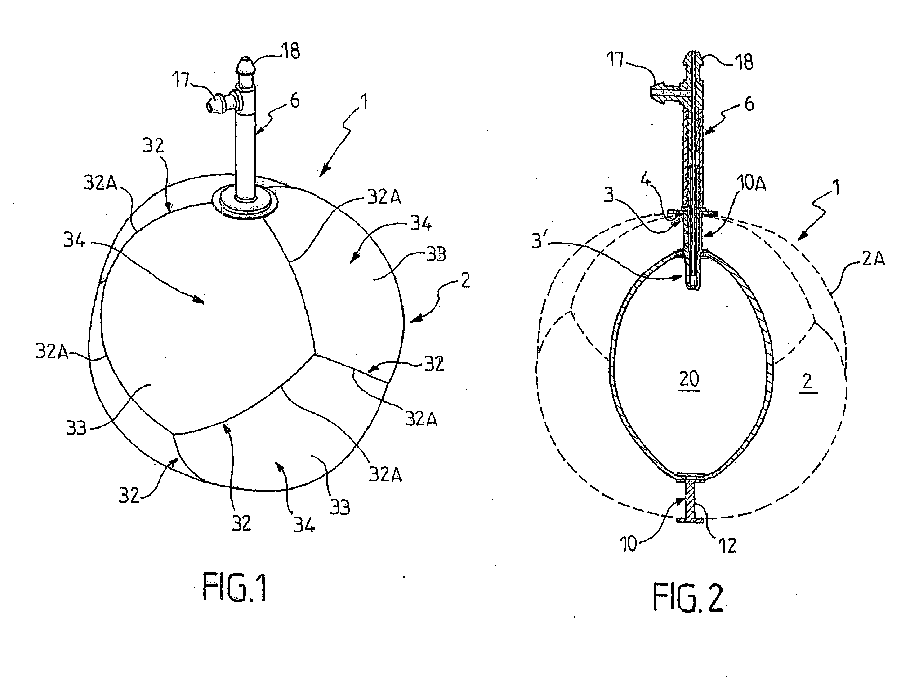

Les figures 1 à 6 montrent un ballon intra-gastrique 1, ainsi que ses détails de réalisation, un tel ballon étant conçu pour le traitement de l'obésité et destiné à être implanté dans l'estomac d'un patient pour réduire le volume de l'estomac, dans la mesure où il occupe une majeure partie de l'espace disponible pour les aliments.

Le ballon intra-gastrique 1 est expansible, c'est-à-dire qu'il est réalisé à base de matériaux souples, par exemple à partir d'élastomère, lui permettant d'occuper d'une part une configuration repliée ou lâche (non représentée aux figures), dans laquelle il occupe un volume restreint favorisant son implantation, et d'autre part, moyennant le recours à un fluide de gonflage, une configuration expansée d'un volume prédéterminé, par exemple de l'ordre de 600 mL, correspondant à son volume d'utilisation, tel qu'illustré en particulier aux figures 1 et 2.

En règle générale, l'implantation du ballon intra-gastrique s'opère de manière classique et bien connue de l'homme du métier par passage dans les voies orales et oesophagiennes sous sa forme repliée ou lâche, l'expansion, la mise en place et le blocage définitif intervenant à la fin de l'opération chirurgicale, lorsque le ballon intra-gastrique 1 est correctement positionné dans l'estomac du patient.

Le ballon intra-gastrique 1 comprend une première poche souple 2 définissant, grâce à ses parois extérieures 2A, un volume interne prédéterminé, ladite première poche souple 2 étant pourvue d'un premier moyen de connexion 3 incluant un orifice 4 et une valve 5, pour recevoir un organe de connexion 6 destiné à être relié à une première source de fluide (non représentée aux figures), en vue d'assurer l'expansion de ladite première poche 2 dans l'estomac par remplissage avec ledit fluide.

Selon une caractéristique importante, et tel qu'illustré aux figures 1 à 6, le ballon intra-gastrique expansible 1 comporte au moins une seconde poche souple 20 de volume également prédéterminé et pourvue d'un second moyen de connexion 3' avec un orifice 4' et une valve 5', ledit second moyen de connexion 3' étant séparé et distinct du premier moyen de connexion 3, de manière à pouvoir être relié à une seconde source de fluide (non représentée aux figures) différente de la première source de fluide.

Grâce à cette disposition et à la séparation et à l'indépendance des deux moyens de connexion 3, 3' correspondant également à une indépendance des deux volumes internes des poches 2, 20, il est possible d'assurer l'expansion et le gonflage de chacune des poches 2, 20 à l'aide de fluides différents, et donc de densités différentes.

En conséquence, pour un même volume global total du ballon intra-gastrique 1, on peut obtenir, à volume égal externe comparable au dispositifs connus, un poids inférieur pour le ballon intra-gastrique 1 conforme à l'invention, en comparaison avec les ballons de l'art antérieur.

Cette disposition permet ainsi de réduire le poids total du ballon intra-gastrique lorsqu'il est implanté dans l'estomac du patient, ce qui améliore sa tolérance par l'organisme et réduit les effets secondaires.

Il est ainsi possible d'assurer le gonflage d'une poche avec du liquide physiologique, l'autre étant gonflée avec un gaz de densité inférieure, par exemple de l'air.

Selon une variante préférentielle, tel qu'illustré aux figures 1 à 6, le ballon intra-gastrique 1 sera avantageusement formé par deux poches 2, 20, de manière à former un ballon bi-poches, étant entendu qu'au sens de l'invention, un nombre supérieur de poches (par exemple 3, 4, voire plus) peut être prévu sans pour autant sortir du cadre de l'invention, chacune étant destinée à être gonflée avec un fluide différent.

Selon une première variante de réalisation (non représentée), les poches 2, 20 peuvent être adjacentes et être reliées par une face commune, la réunion, par exemple par collage des poches, formant le ballon.

Selon une autre version particulièrement avantageuse, et tel qu'illustré aux figures 1 à 6, le ballon intra-gastrique 1 comporte au moins une seconde poche 20 qui est disposée à l'intérieur de la première poche 2, et qui est donc d'un volume externe inférieur, la première poche 2 étant donc de dimensions supérieures, au moins à l'état expansé.

Selon cette variante préférentielle, la seconde poche 20 forme donc une poche interne de forme générale identique, voire différente de la poche 2 qui forme la poche principale.

Selon cette réalisation, la poche 20 sera, de manière préférentielle, remplie avec un gaz, par exemple de l'air, alors que la première poche 2 sera remplie avec un liquide, par exemple de l'eau physiologique. La seconde poche 20 étant disposée, de manière avantageuse sensiblement concentrique à la première poche 2, et donc entourée sur sensiblement toute sa surface extérieure par le liquide de la poche 2, on obtient une bonne étanchéité de la poche 20, ce qui réduit les risques de fuite du gaz qui y est contenu.

Selon les variantes préférentielles de réalisation illustrées aux figures 1 à 6, les premier et second moyens de connexion 3, 3' sont sensiblement alignés, de manière que leurs orifices respectifs 4, 4' puissent recevoir facilement un organe de connexion commun 6. Cette disposition a pour objet de faciliter grandement l'opération délicate et essentielle de gonflage et d'expansion des deux poches 2, 20, en réduisant le nombre de manipulations nécessaires, ainsi que le nombre d'instruments requis.

Selon la variante préférentielle formée par un ballon 1 bi-poches, le maintien d'un espacement entre les poches 2, 20 est assuré à l'aide de moyens d'immobilisation 10 chargés de maintenir respectivement et à distance l'une de l'autre les deux poches 2, 20.

Selon une variante préférentielle de l'invention, les moyens d'immobilisation 10 sont formés par des entretoises maintenant et fixant les deux poches 2, 20 à distance l'une de l'autre.

Avantageusement, le ballon intra-gastrique 1 comportera deux entretoises 10, sensiblement diamétralement opposées l'une à l'autre par rapport au centre commun des deux poches (figure 2).

A titre de variante, il est bien évidemment possible d'envisager un nombre supérieur de moyens d'immobilisation. En particulier, on pourrait envisager une série de quatre, voire six entretoises (ou moyens équivalents), réparties angulairement, de manière régulière ou non entre les deux poches 2, 20.

Avantageusement, les entretoises 10 sont formées par au moins une, et de préférence deux embases 11, reliées entre elles par une jambe 12 formant l'entretoise proprement dite, l'embase 11 ou chaque embase 11 étant fixée, par exemple par collage, sur les parois des poches 2, 20.

Selon la variante de réalisation préférentielle illustrée aux figures 1 à 3, le premier et le deuxième moyens de connexion 3, 3' sont communs aux deux poches 2, 20, et formés par l'une des entretoises 10 qui forme donc un moyen de connexion commun 10A. Cette disposition simplifie grandement la fabrication et le montage du ballon intra-gastrique 1, tout en lui assurant une bonne robustesse.

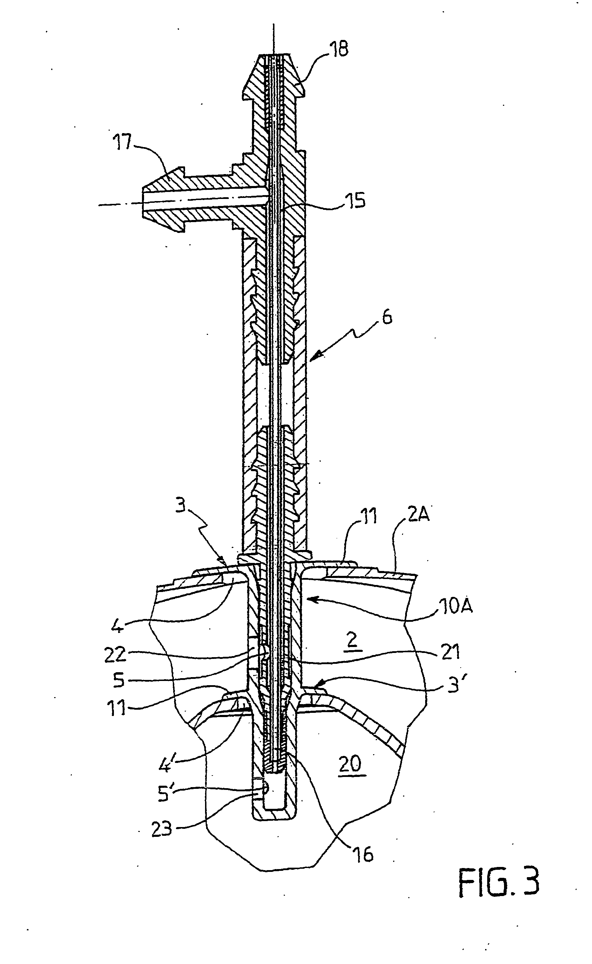

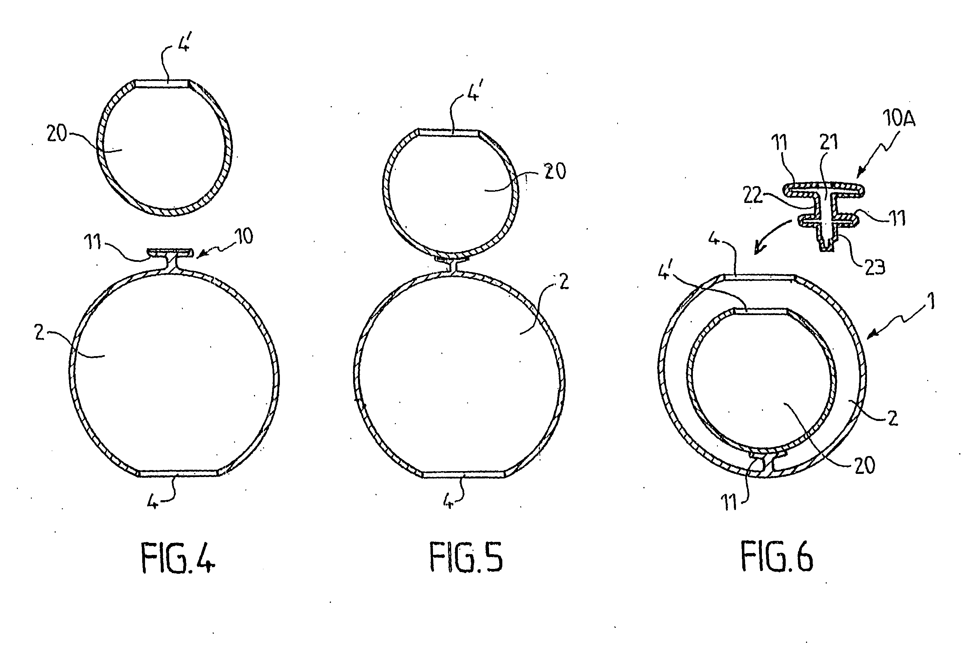

L'entretoise 10 formant le moyen de connexion commun 10A est illustré en détails à la figure 3 et à la figure 6.

Le moyen de connexion commun 10A comporte un conduit central creux 21 formé par la jambe de l'entretoise, ledit conduit 21 comportant deux perçages 22, 23, chacun ménagé respectivement à une hauteur telle pour être en regard du volume interne de la première et de la deuxième poches 2, 20, chacun étant associé à une valve, respectivement 5, 5', ledit conduit 21 étant apte à recevoir l'organe de connexion 6, destiné à assurer le remplissage différencié de chaque poche 2, 20 avec un fluide de remplissage distinct.

De manière connue, le moyen de connexion commun 10A est réalisé dans un matériau élastomère relativement mou, et donc de dureté faible, de telle manière que les perçages 22, 23 sont réalisés sous la forme de simples incisions à deux étages différents. Grâce à la dureté faible du matériau élastomère, les valves 5, 5' sont en réalité constituées par le perçage ou l'incision 22, 23, qui permettent d'assurer l'étanchéité de chaque poche dès la fin du remplissage par simple élasticité.

Tel qu'illustré en particulier à la figure 1, le ballon intra-gastrique pourra comporter une paroi extérieure 2A formée de facettes ou alvéoles réparties de manière régulière ou non sur la totalité de la surface du ballon 1.

Cette particularité de conception, qui peut être totalement indépendante de la présence ou de l'absence d'une ou plusieurs poche(s) dans le ballon intra-gastrique 1, permet notamment de multiplier la probabilité de zones de contact périphériques avec les parois de l'estomac du patient. Ceci augmente donc la possibilité et la probabilité de gêner de manière durable le passage des aliments, ce qui a tendance à prolonger également la sensation de satiété.

La forme alvéolée peut être obtenue par une série d'alvéoles jointives sur toute la surface de la poche principale 2, ou au contraire par une série d'alvéoles non jointives, la surface inter-alvéole correspondant par exemple à une portion de sphère.

On peut protéger de manière individuelle l'organe de connexion 6 destiné à assurer l'interface entre le ballon intra-gastrique 1 conforme à l'invention et une ou plusieurs source(s) de fluide (non représentées aux figures), qui fournissent le fluide nécessaire au gonflage et à l'expansion dudit ballon.

A cette fin, on peut utiliser un dispositif chirurgical pour le traitement de l'obésité destiné à expanser un ballon intra-gastrique multi-poches, et en particulier bi-poches, tel qu'illustré à la figure 2.

Selon cette configuration, le dispositif chirurgical comporte un organe de connexion tubulaire 6 apte à être inséré dans le conduit central creux 21 du moyen de connexion commun 10A du ballon intra-gastrique, ledit organe 6 étant pourvu de deux canaux indépendants 15, 16 débouchant vers une extrémité, respectivement en regard des deux perçages 22, 23 du conduit central creux 21, lesdits canaux 15, 16 étant reliés par l'autre extrémité à deux embouts 17, 18 indépendants l'un de l'autre et aptes à être connectés chacun séparément à une source de fluide de remplissage distincte.

Selon la variante de réalisation préférentielle illustrée à la figure 3, les canaux indépendants 15, 16 sont concentriques. A titre de variante, ils peuvent néanmoins être non concentriques et séparés, et s'étendre parallèlement l'un de l'autre et à distance dans l'organe de connexion tubulaire 6.

En utilisation, une fois le ballon intra-gastrique 1 implanté dans sa forme dégonflée au sein de l'estomac du patient par voie oesophagienne, à l'aide par exemple d'un endoscope, l'organe de connexion tubulaire 6 ayant été inséré, préalablement ou non à l'implantation, dans le conduit central 21, l'opération de gonflage et d'expansion des poches 2, 20 peut débuter.

Au préalable, chaque embout 17, 18 a été connecté et raccordé à une source de fluide de remplissage distincte, à savoir par exemple pour l'embout 17 à une source de liquide (liquide physiologique), et pour l'embout 18 à une source de gaz (air par exemple). L'expansion de chacune des poches 2, 20 peut donc s'effectuer de manière simultanée, à l'aide par exemple de seringues, la pression de chaque fluide étant suffisante pour assurer la déformation des incisions constituant les perçages 22, 23.

Lorsque le volume prédéterminé de fluide requis est atteint, le débit d'admission de chaque fluide est arrêté, ce qui, compte tenu de l'élasticité du matériau du moyen de connexion commun 10A, permet à chaque perçage 22, 23 de reprendre sa position initiale de fermeture correspondant à leur position d'étanchéité. Il n'y a ainsi aucun risque de fuite ou de mélange des fluides de chacune des poches 2, 20.

L'organe de connexion tubulaire 6 est ensuite extrait du conduit central creux 21, et donc du ballon intra-gastrique 1, par simple traction longitudinale ou axiale.

Le ballon intra-gastrique 1 peut être obtenu à l'aide de tout procédé classique de fabrication connu faisant intervenir des étapes de trempage d'un moule sensiblement sphérique dans un bain de matériaux élastomères, par exemple à base d'un mélange de silicone et de xylène.

Néanmoins, le ballon intra-gastrique conforme à l'invention sera avantageusement obtenu à l'aide d'un procédé d'injection de matières élastomères à base de silicone dans des moules pour obtenir séparément, au cours d'une première étape d'injection, chacune des deux poches 2, 20 pourvues respectivement de leurs orifices 4, 4', tel qu'illustré à la figure 4.

Selon cette première étape, le procédé de fabrication conforme à l'invention est donc un procédé dans lequel on assure la fabrication des première et deuxième poches par injection d'un matériau élastomère dans un moule, pour obtenir deux poches avec chacune un orifice 4, 4', la première poche étant de dimensions supérieures à la seconde poche, de manière que cette dernière puisse être insérée dans la première poche avec une distance périphérique suffisante.

Le procédé de fabrication consiste ensuite, au cours de l'étape de fabrication par injection d'élastomère de la première poche ou après, c'est-à-dire pendant une étape ultérieure, à assurer la mise en place, sur la face externe de la première poche, sensiblement à l'opposé de l'orifice 4, d'une entretoise 10 présentant une embase externe libre 11 tournée vers l'extérieur de ladite première poche.

La mise en place de l'entretoise 10 peut être faite directement au cours de l'étape de fabrication par injection d'élastomère, l'entretoise 10 étant donc réalisée directement également par injection d'élastomère.

A titre de variante préférentielle, l'entretoise 10 peut être réalisée indépendamment au cours d'une étape d'injection distincte avec un matériau élastomère différent. Dans ce cas, l'entretoise 10 est solidarisée par collage sur la face externe de la poche 2, sensiblement à l'opposé et au droit de l'orifice 4.

Par la suite, tel qu'illustré à la figure 5, on assure le collage relatif des deux poches 2, 20 au niveau de l'embase externe libre 11 sur la face externe de la seconde poche 20, à un endroit qui est situé sensiblement à l'opposé ou à l'aplomb de l'orifice correspondant 4'.

Puis on réalise le retournement de la première poche 2, à la manière du retournement d'un manchon ou d'une chaussette, en faisant pénétrer par son orifice 4 la seconde poche 20, de manière à obtenir, tel qu'illustré à la figure 6, une disposition dans laquelle la seconde poche 20 est située à l'intérieur de la poche 2 et sensiblement concentrique à cette dernière, leurs deux orifices 4, 4' étant alignés axialement et situés au droit l'un de l'autre.

Selon une étape complémentaire, on assure ensuite la mise en place du moyen de connexion commun 10A qui est fabriqué séparément au cours d'une étape séparée de moulage par injection d'élastomère.

Selon cette étape complémentaire, on assure la mise en place, par les deux orifices 4, 4' sensiblement alignés des première et deuxième poches 2, 20, du moyen de connexion commun 10A aux deux poches, puis le collage dudit moyen commun 10A par l'intermédiaire de ses deux embases ou pieds d'ancrage 11 sur chacune des portions annulaires entourant les orifices 4, 4'.

Le procédé de fabrication par injection d'élastomère s'avère ainsi d'une mise en oeuvre simple et rapide réduisant les contraintes industrielles.

On peut prévoir également, de façon totalement indépendante, un ballon intra-gastrique pour le traitement de l'obésité destiné à être implanté dans l'estomac d'un patient pour réduire le volume de l'estomac, ledit ballon comprenant une paroi extérieure destinée à venir en contact avec la paroi de l'estomac, le ballon séparant l'estomac en une zone amont et une zone aval en vue de constituer une barrière au passage des aliments entre ces deux zones, la paroi extérieure étant conformée pour définir, en coopération avec la paroi de l'estomac, des canaux de passage de la zone amont vers la zone aval pour les aliments.

Il apparaît en effet que si le facteur principal influant sur la perte de poids pour le patient lorsque ce dernier est traité par un ballon intra-gastrique est le volume occupé par ce ballon dans l'estomac du patient, d'autres facteurs sont à prendre en considération. Ainsi, le mode de contact du ballon sur les parois de l'estomac, que ce soit d'un point de vue de la texture du ballon ou de la géométrie du contact entre le ballon et la paroi de l'estomac, peut jouer un rôle important dans l'efficacité du traitement par ballon intra-gastrique, ou du moins dans le confort d'un tel traitement.

Il s'avère en effet qu'une portion de la surface du ballon, qui correspond généralement sensiblement à une bande circonférentielle, vient se caler contre la paroi gastrique lorsque le ballon est mis en place dans l'estomac. Le ballon forme ainsi une barrière entre une zone amont de l'estomac, en communication avec l'oesophage, et une zone aval de l'estomac, en communication avec le reste du tractus digestif. Lorsque la bande périphérique de contact entre le ballon et la paroi de l'estomac est large, ce qui correspond à une interface de contact importante entre le ballon et la paroi de l'estomac, le ballon constitue une barrière extrêmement difficile à franchir pour les aliments en provenance de la zone amont et qui doivent passer dans la zone aval pour accéder ensuite au reste du tractus digestif. Un tel cas est notamment rencontré lorsqu'un ballon sphérique à surface lisse, ou du moins régulière, est utilisé. Dans ce cas, une bande périmétrique plus ou moins large de la surface externe du ballon vient épouser les parois de l'estomac, ce qui interdit le passage et donc la digestion des aliments en provenance de l'oesophage. Lorsqu'une quantité importante d'aliments s'est accumulée dans la zone amont, cela induit une charge mécanique sur le ballon qui provoque des décollements localisés de la paroi du ballon et de la paroi de l'estomac, autorisant ainsi le passage des aliments.

Le ballon fonctionne ainsi à la manière d'un clapet, ce qui perturbe fortement la digestion des aliments et peut provoquer des aigreurs d'estomac particulièrement incommodantes pour le patient.

Afin de surmonter cette difficulté, il a été proposé, tel que cela est décrit dans le document US-4 694 827, un ballon intra-gastrique dont la surface extérieure est pourvue de lobes, lesquels sont supposés assumer une fonction double, à savoir celle de définir des canaux entre la surface du ballon et la paroi de l'estomac, tout en minimisant le contact entre la paroi de l'estomac et le ballon.

Une telle conception, si elle permet effectivement de réaliser un contact sensiblement tangentiel entre l'enveloppe du ballon et la paroi de l'estomac, ce qui minimise le risque de traumatisme de la paroi gastrique par le ballon, autorise cependant un transit extrêmement facile et rapide des aliments vers le reste du circuit digestif, ce qui ne permet pas de procurer au patient une sensation de satiété prolongée.

Or, une partie de l'efficacité du traitement réside dans l'effet barrière que procure le ballon, c'est-à-dire dans la difficulté que rencontrent les aliments à passer de la zone amont vers la zone aval de l'estomac, ce qui prolonge le sentiment de satiété éprouvé par le patient.

On peut prévoir un nouveau ballon intra-gastrique dont la conformation, qui permet un contact minimisé du ballon avec les parois de l'estomac, garantit le passage des aliments de la zone amont vers la zone aval, tout en assurant un temps de passage suffisamment long pour que la sensation de satiété induite par le ballon soit optimisée.

On peut prévoir un nouveau ballon intra-gastrique présentant un caractère atraumatique.

On peut prévoir un nouveau ballon intra-gastrique particulièrement simple et peu onéreux à fabriquer.

Les objets assignés sont atteints à l'aide d'un ballon intra-gastrique pour le traitement de l'obésité, destiné à être implanté dans l'estomac d'un patient pour réduire le volume de l'estomac, ledit ballon comprenant une paroi extérieure destinée à venir en contact avec la paroi de l'estomac, le ballon séparant l'estomac en une zone amont et une zone aval en vue de constituer une barrière au passage des aliments entre ces deux zones, la paroi extérieure étant conformée pour définir, en coopération avec la paroi de l'estomac, des canaux de passage de la zone amont vers la zone aval pour les aliments, caractérisé en ce que la paroi extérieure est conformée pour que les canaux forment un réseau ramifié en plus de deux points, de façon à constituer un parcours arborescent pour les aliments passant de la zone amont à la zone aval.

D'autres objets et avantages apparaîtront mieux à la lecture de la description détaillée qui suit, ainsi qu'à l'aide des dessins annexés, donnés à titre purement illustratif et non limitatif, dans lesquels :

- La figure 1 illustre, selon une vue en perspective, un ballon intra-gastrique selon une première variante de réalisation.

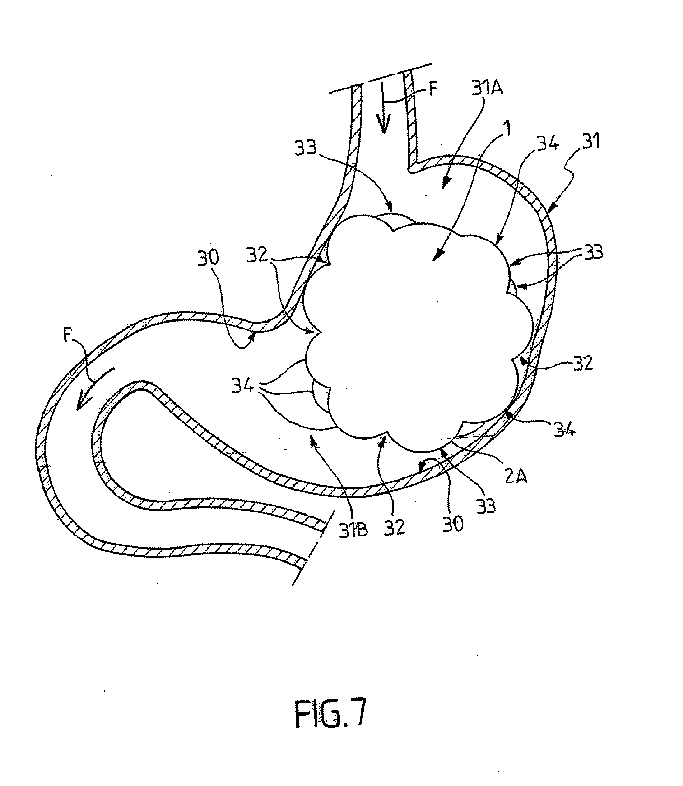

- La figure 7 illustre, selon une vue en coupe schématique, un ballon intra-gastrique calé contre les parois de l'estomac.

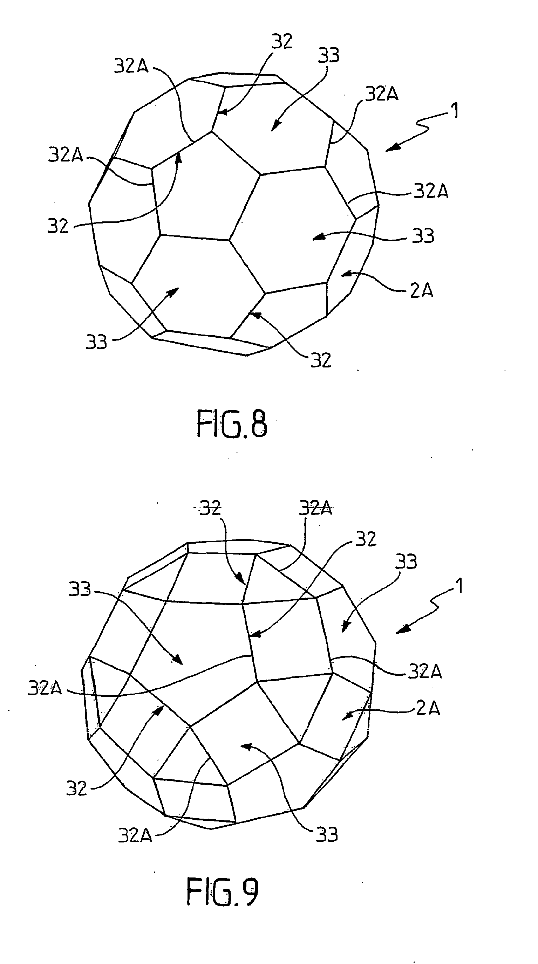

- La figure 8 illustre, selon une vue en perspective, un deuxième mode de réalisation d'un ballon intra-gastrique, dans une position d'expansion incomplète.
- La figure 9 illustre, selon une vue en perspective, un troisième mode de réalisation d'un ballon intra-gastrique conforme à l'invention, dans une position d'expansion incomplète.

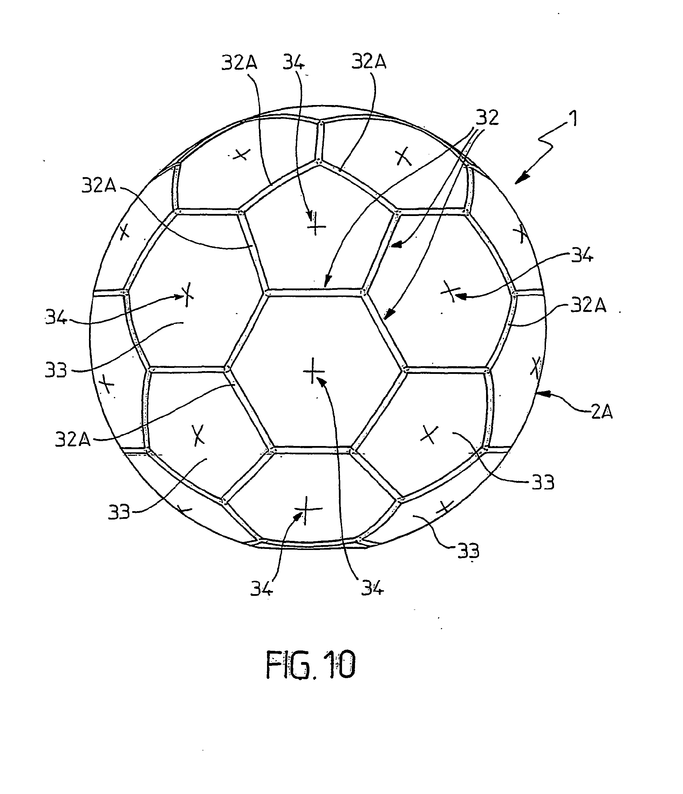

- La figure 10 illustre, selon une vue en perspective, le ballon de la figure 8 en position d'expansion maximale.

Les figures 1 et 7 à 10 montrent un ballon intra-gastrique 1. Un tel ballon est conçu pour le traitement de l'obésité et est destiné à être implanté dans l'estomac d'un patient pour réduire le volume de l'estomac, dans la mesure où il occupe une majeure partie de l'espace disponible pour les aliments.

Dans ce qui suit, il sera fait référence à un ballon constitué d'une poche souple expansible par remplissage de fluide, la poche étant constituée d'une enveloppe réalisée à base de matériau souple, par exemple à partir d'élastomère, et dont la face externe forme une paroi extérieure 2A destinée à venir en contact avec la paroi 30 de l'estomac 31. Une telle réalisation permet au ballon d'occuper d'une part une configuration repliée ou lâche (non représentée aux figures), dans laquelle il occupe un volume restreint favorisant son implantation, et d'autre part, moyennant le recours à un ou plusieurs fluides de gonflage (par exemple un liquide et/ou un gaz), une configuration expansée d'un volume prédéterminé, par exemple de l'ordre de 600 mL, correspondant à son volume d'utilisation, tel qu'illustré en particulier aux figures 1, 7 et 10. Entre la configuration repliée et la configuration expansée, le ballon 1 passe par des configurations de gonflement intermédiaire représentées aux figures 8 et 9, où le ballon est proche de son volume nominal d'utilisation, mais ne l'a pas tout à fait encore atteint (gonflement incomplet).

Il est cependant tout à fait envisageable, sans pour autant sortir du cadre de l'invention, que le ballon intra-gastrique 1 conforme à l'invention présente une structure qui ne présente pas un caractère expansible, mais plutôt un caractère rigide ou semi-rigide. Il est également envisageable, sans sortir du cadre de l'invention, que le ballon 1 soit constitué d'une structure pliable dont l'expansion ne nécessite pas de fluide, mais est réalisée par un effet élastique ou grâce à la mise en oeuvre de structures à mémoire de forme.

Le ballon 1 occupe donc un volume suffisamment important au sein de l'estomac pour qu'il soit en mesure de venir se caler contre la paroi 30 de l'estomac 31.

De cette façon, le ballon 1 sépare l'estomac 31 en une zone amont 31 A et une zone aval 31 B. La zone amont 31 A se situe ainsi à l'amont du ballon 1 dans le sens du flux digestif F des aliments ingérés par le patient, tandis que la zone aval 31 B se trouve à l'aval dudit ballon, dans le sens du flux digestif F des aliments. La zone 31 A communique donc avec l'oesophage, tandis que la zone 31 B est en communication avec le reste du tractus digestif, c'est-à-dire les intestins.

Le ballon 1 constitue ainsi une barrière au passage des aliments de la zone amont 31 A vers la zone aval 31 B.

L'optimisation de l'efficacité du ballon réside, outre dans le volume qu'il occupe dans l'estomac et qui limite de fait la place disponible pour les aliments, dans le contrôle du franchissement par les aliments de la barrière constituée par le ballon 1.

A cette fin, la paroi extérieure 2A du ballon 1 est conformée pour définir, en coopération avec la paroi de l'estomac 30, des canaux de passage 32 de la zone amont 31 A vers la zone aval 31 B pour les aliments.

Ces canaux 32 assurent une fonction de franchissement de la barrière constituée par le ballon 1, et de guidage des aliments solides et/ou liquides de la zone amont 31A vers la zone aval 31B.

La paroi extérieure 2A est conformée pour que les canaux 32 soient interconnectés de façon à former un réseau ramifié en plus de deux points, et constituer ainsi un parcours arborescent pour les aliments passant de la zone amont 31 A vers la zone aval 31 B.

Par « *réseau ramifié en plus de deux points* », on désigne ici le fait que des subdivisions ou jonctions de canal sont prévues en au moins trois points du réseau de canaux.

Le concept général vise donc d'une part à assurer de façon sensiblement garantie, grâce aux canaux 32, le passage des aliments de la zone amont 31A vers la zone aval 31 B, ce qui permet d'éviter les inconvénients liés à un effet « *clapet* » du ballon qui se produirait si ce dernier générait un contact sensiblement étanche avec la paroi 30 de l'estomac 31. D'autre part, tout en assurant un passage certain pour les aliments, le ballon intra-gastrique 1 permet cependant de générer un parcours tortueux à chemins multiples pour la progression des aliments dans l'entre-paroi estomac/ballon, grâce à la formation d'un réseau ramifié en plus de deux points par les canaux 32. Les aliments sont donc assurés de passer sensiblement continûment de la zone amont 31 A vers la zone aval 31 B, mais selon un rythme lent conféré par le caractère ramifié, c'est-à-dire subdivisé, du réseau de canaux 32 de passage entre la paroi 30 de l'estomac et la paroi extérieure 2A du ballon 1. Un tel agencement permet d'augmenter la durée globale de digestion des aliments, et donc de prolonger l'effet de satiété, en s'affranchissant sensiblement de tout effet « clapet ».

Dans ce qui suit, il sera fait référence à un ballon présentant une forme sensiblement arrondie, ou du moins s'inscrivant sensiblement dans une sphère. Une telle configuration n'est cependant donnée qu'à titre d'exemple, et la forme générale du ballon 1 pourra ainsi éventuellement se rapprocher d'une ellipsoïde, ou encore d'une forme ovoïde par exemple.

Avantageusement, le ballon intra-gastrique 1 comprend une pluralité de bosses 33 agencées les unes par rapport aux autres, de telle sorte que les canaux 32 de passage des aliments de la zone amont 31 A vers la zone aval 31 B soient délimités d'une part par les espaces ou interstices séparant les bosses 33 les unes des autres et, d'autre part, par la paroi 30 de l'estomac en contact avec le sommet 34 desdites bosses 33.

Par « bosse », on désigne ici une protubérance ou une excroissance de forme convexe sensiblement arrondie ou régulière. Chaque bosse 33 présente un sommet 34 destiné à venir en contact avec la paroi gastrique 30. Le contact entre ladite paroi 30 de l'estomac 31 et le ballon 1 s'effectue ainsi selon une pluralité de contacts sensiblement ponctuels ou tangentiels, ce qui minimise la surface totale de contact entre la paroi gastrique 30 et le ballon 1, et limite ainsi considérablement le risque traumatique. Les bosses 33 sont agencées suffisamment proches les unes des autres pour définir à leur base des rigoles ou gorges qui sont fermées par la paroi 30 de l'estomac 31, pour former des canaux 32 d'acheminement et de guidage des aliments liquides ou solides de la zone amont 31 A vers la zone aval 31 B.

Les bosses 33 sont également disposées de façon à ce que chaque rigole se subdivise en deux bras ou plus, et ce, en plus de deux points. Cette subdivision ou ramification crée un chemin de passage arborescent pour les aliments, ce qui contribue à augmenter leur temps de passage entre la zone amont 31 A et la zone aval 31 B par rapport à un chemin direct, tel que celui décrit dans le document US-4 694 827.

Avantageusement, et tel que cela représenté aux figures 8 à 10, chaque bosse 33 fait saillie d'une base sensiblement polygonale. De façon préférentielle, les bases sensiblement polygonales sont jointives par leurs côtés 32A sur au moins une partie, et de préférence sur la totalité, de la surface de la paroi extérieure 2A.

La paroi externe 2A du ballon peut ainsi présenter une multitude de facettes ou alvéoles réparties de manière régulière ou non sur tout ou partie de sa surface, chaque facette formant la base d'une bosse 33, dont le sommet 34 est sensiblement à l'aplomb du centre de la facette. Les facettes peuvent être strictement planes, ou plus ou moins courbées ou bombées, notamment après gonflage maximum du ballon, tel que cela est représenté aux figures 1 et 10.

Selon les modes de réalisation présentés aux figures 8 à 10, les bases polygonales dont sont issues les bosses 33 peuvent être de nature différente et présenter des formes et/ou des nombres de côtés variables.

De façon préférentielle, les bases sont agencées les unes par rapport aux autres sensiblement selon un motif de polyèdre, régulier ou semi-régulier.

Il est ainsi envisageable que les bases polygonales soient agencées selon un motif de dodécaèdre ou d'icosaèdre.

De façon préférentielle, les bases sont agencées selon un motif de polyèdre semi-régulier (polyèdre archimédien) tel que le dodécaèdre tronqué, l'icosaèdre tronqué (représenté aux figures 8 et 10), l'icosidodécaèdre, le petit rhombicuboctaèdre, le grand rhombicuboctaèdre, le petit rhombicosidodécaèdre (représenté à la figure 9) ou encore le grand rhombicosidodécaèdre, cette liste n'étant nullement limitative.

De façon préférentielle, l'enveloppe du ballon est formée par l'assemblage des bases polygonales selon leurs côtés 32A, le coeur 33 de chaque base, délimité par les côtés 32A, présentant une souplesse ou une déformabilité supérieure à celle desdits côtés 32A, de telle sorte que lors du gonflement par remplissage de fluide de la poche formée par l'enveloppe, le coeur 33 de chaque base se déforme plus que les bords formant les côtés 32A de chaque base, ce qui a pour effet de générer des protubérances formant les bosses 33, le sommet 34 de ces protubérances se situant sensiblement à la verticale du voisinage du centre du polygone formé par les côtés 32A de chaque base.

Chaque base forme ainsi une facette à partir de laquelle est générée une bosse 33.

On peut ainsi envisager de réaliser l'enveloppe en un matériau élastomère de façon à ce que le coeur 33 de chaque base ou facette soit d'une épaisseur plus réduite que celle des côtés 32A de chacune des bases ou facettes, ce qui procure un différentiel de souplesse entre le coeur 33 et les côtés 32A de chaque base ou facette. Ainsi, sous l'effet d'une sollicitation mécanique, en l'occurrence une pression interne de gonflage, les zones d'épaisseur importante, c'est-à-dire les côtés 32A, ne vont pas ou très peu se déformer, tandis que les zones d'épaisseur moindre, c'est-à-dire les coeurs de facette 33, vont se déformer de façon plus importante pour former les bosses 33, ce qui permet d'obtenir une surface de paroi extérieure 2A boursouflée ou bosselée, c'est-à-dire formée d'une alternance de surfaces convexes et concaves, un réseau arborescent de rigoles étant créé de ce fait entre les bosses 33.

De façon alternative, il est envisageable de réaliser l'enveloppe d'un ballon conforme à l'invention en associant un tissu, c'est-à-dire un textile tissé ou une simple grille ou treillis, avec un film élastomérique formant le coeur de chaque base ou facette, les mailles dudit tissu formant les côtés des bases dont sont issues les bosses 33 et présentant une déformabilité inférieure à celle du film élastomérique, de façon à créer des bosses 33 au centre de chaque maille. L'association du tissu et du film élastomérique pourra par exemple être réalisée en noyant le tissu de renfort au sein d'une matrice en élastomère, par exemple en silicone. Lors du gonflage de l'enveloppe, le textile agira comme renfort en autorisant seulement la déformation du matériau élastomérique au coeur de chaque maille formant facette.

Selon une autre variante de réalisation non représentée, il est tout à fait envisageable que la paroi extérieure 2A présente une forme sensiblement lisse et sphérique parsemée d'excroissances ayant la forme de calottes sphériques, lesdites excroissances étant distribuées sur l'ensemble de la surface de la paroi extérieure 2A, de façon suffisamment proche les unes des autres, pour définir des rigoles, qui participeront, en coopération avec la paroi 30 de l'estomac, à la formation des canaux 32.

Ainsi, lorsque le ballon 1 est en position dans l'estomac, calé latéralement et périphériquement contre la paroi de ce dernier, les aliments présents dans la zone amont 31A vont emprunter un premier canal 32, puis rencontrer un premier obstacle formé par une bosse 33, qui subdivise le canal 32 en au moins deux canaux, et ainsi de suite. Cet agencement permet un passage calibré et contrôlé des aliments vers le reste du circuit digestif.

De façon totalement indépendante des caractéristiques techniques présentées précédemment, l'invention concerne également un ballon intra-gastrique pour le traitement de l'obésité, destiné à être implanté dans l'estomac d'un patient pour réduire le volume de l'estomac, ledit ballon comprenant une enveloppe souple délimitant un volume interne prédéterminé, ladite enveloppe souple étant réalisée en un matériau élastomère.

L'invention concerne également de façon indépendante un procédé de fabrication d'un ballon intra-gastrique pour le traitement de l'obésité, ledit ballon étant destiné à être implanté dans l'estomac d'un patient pour réduire le volume de l'estomac.

Les ballons intra-gastriques, réalisés sous la forme d'une poche expansible monobloc en silicone, généralement de forme sphérique, sont bien connus. Ils sont généralement fabriqués par trempage. Le procédé de trempage consiste à tremper un noyau présentant la forme souhaitée pour le ballon (par exemple sphère, ovoïde, ellipsoïde) dans un bain de silicone dispersé dans un solvant, de faire sécher le film formé à la surface du noyau par ce trempage, puis de le démouler du noyau.

Un tel procédé de fabrication, s'il donne généralement satisfaction, présente néanmoins de nombreux inconvénients.

En effet, du fait de la présence de solvant inflammable et toxique dans le bain de trempage, il est nécessaire d'utiliser des équipements spéciaux, tant au niveau des machines que du génie civil (salle anti-déflagrante) pour garantir la santé et la sécurité des opérateurs. De plus, ce procédé est particulièrement délicat à mettre en oeuvre, car il nécessite un pilotage précis de la fluidité du bain de trempage, ce qui requiert une surveillance et une ré-alimentation constante en solvant, ce dernier étant généralement très volatile.

Ce procédé nécessite donc un personnel particulièrement qualifié.

Par ailleurs, il est généralement nécessaire de procéder à plusieurs étapes de trempage afin d'obtenir, par itération, l'épaisseur finale d'enveloppe souhaitée. Entre chaque étape successive de trempage, on doit veiller à assurer l'évaporation du solvant, afin de permettre la réticulation de la couche de matière déposée sur le noyau. Dès lors, le procédé de fabrication par trempage s'avère particulièrement long, puisqu'il nécessite des séquences d'opérations de natures différentes (trempage, déplacement, passage au four pour évaporation du solvant, etc.), justifiant l'utilisation d'un carrousel de convoyage, qui font que le temps de cycle d'obtention d'un ballon est de l'ordre de la demi-journée. En outre, le fait de réaliser le ballon par un empilement de couches distinctes successives introduit un risque d'imperfection de cohésion entre deux couches, susceptible de nuire au caractère unitaire recherché pour le ballon.

De surcroît, le procédé par trempage ne permet généralement pas de maîtriser de façon précise l'épaisseur de l'enveloppe du ballon. Les ballons obtenus par trempage, s'ils donnent généralement satisfaction, souffrent cependant d'une précision dimensionnelle insuffisante, qui peut conduire à des sur-épaisseurs en certaines zones du ballon, ce qui augmente les coûts de production du ballon, ou à des sous-épaisseurs en d'autres endroits, ce qui peut engendrer une fragilisation du ballon.

Enfin, le champ d'application de ce procédé est d'autant plus limité qu'il ne permet pas de réaliser des variations contrôlées d'épaisseur et/ou de forme sur une même pièce. Dès lors, la conception d'un ballon intra-gastrique devant être fabriqué par ce procédé reste conditionnée par l'écoulement d'un fluide sur un noyau, ce qui réduit considérablement les possibilités d'associer formes, dimensions, et en définitive, fonctionnalités, sur une même pièce.

Les objets assignés à l'invention visent en conséquence à porter remède aux différents inconvénients énumérés précédemment et à proposer un nouveau ballon intra-gastrique présentant une résistance et une régularité améliorée.

Un autre objet de l'invention vise à proposer un nouveau procédé de fabrication d'un ballon intra-gastrique permettant la fabrication de ballons de façon plus sûre, plus simple et pour un coût moindre.

Un autre objet de l'invention vise à proposer un nouveau procédé de fabrication d'un ballon intra-gastrique qui permet de fabriquer des ballons de façon rapide avec une excellente régularité dimensionnelle.

Un autre objet de l'invention vise à proposer un nouveau procédé de fabrication d'un ballon intra-gastrique dont le nombre d'étapes est réduit.

Les objets assignés à l'invention sont atteints à l'aide d'un ballon intra-gastrique pour le traitement de l'obésité destiné à être implanté dans l'estomac d'un patient pour réduire le volume de l'estomac, ledit ballon comprenant une enveloppe souple délimitant un volume interne prédéterminé, ladite enveloppe souple étant réalisée en un matériau élastomère, caractérisé en ce que la tolérance dimensionnelle de l'épaisseur nominale de l'enveloppe est comprise entre 1 % et 20%.

Les objets assignés à l'invention sont également atteints à l'aide d'un procédé de fabrication d'un ballon intra-gastrique pour le traitement de l'obésité, ledit ballon étant destiné à être implanté dans l'estomac d'un patient pour réduire le volume de l'estomac, caractérisé en ce qu'il comprend une étape d'injection dans laquelle on injecte un matériau élastomère dans un moule, pour obtenir une poche souple destinée à former l'enveloppe du ballon.

D'autres objets et avantages de l'invention apparaîtront mieux à la lecture de la description qui suit, ainsi qu'à l'aide des dessins annexés donnés à titre purement illustratif et non limitatif, dans lesquels :

- La figure 1 illustre, selon une vue en perspective, un ballon intra-gastrique

- La figure 10 illustre, selon une vue en perspective, un ballon un deuxième mode de réalisation.

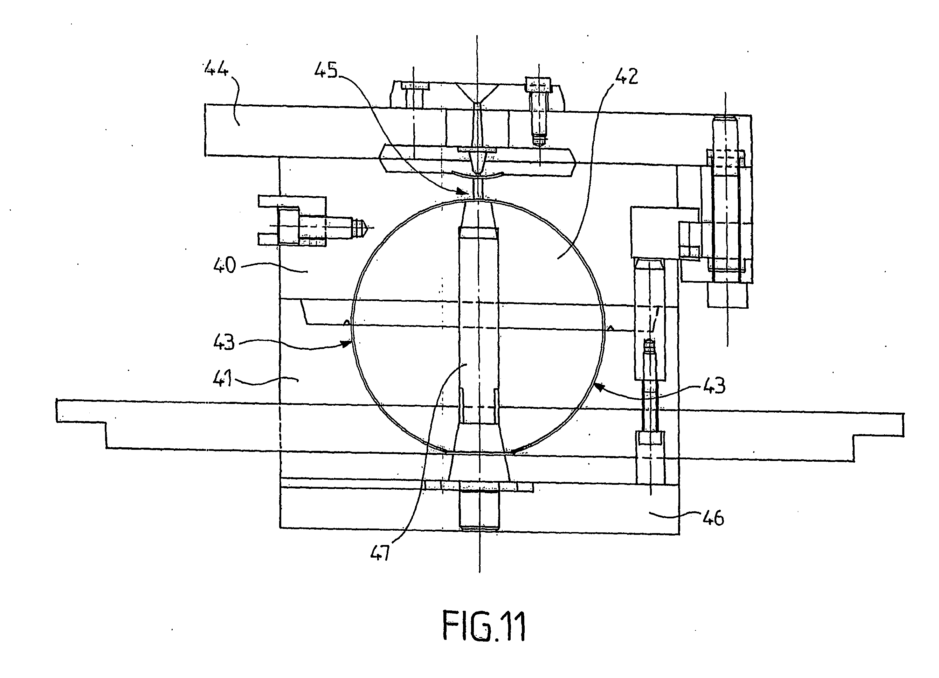

- La figure 11 illustre, selon une vue de côté, un moule en position fermée permettant la mise en oeuvre du procédé conforme à l'invention.

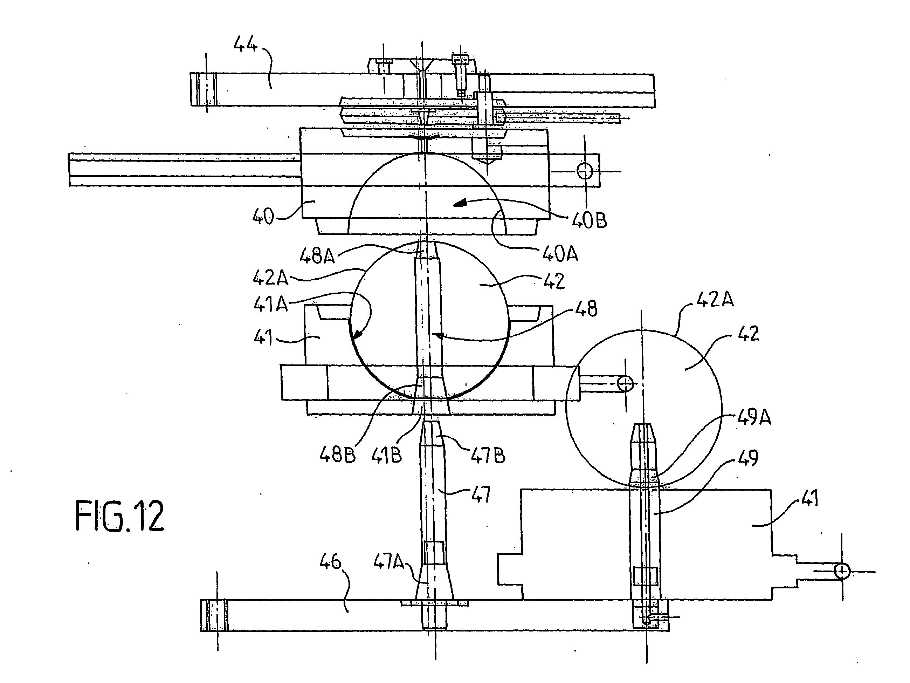

- La figure 12 illustre, selon une vue de côté, le moule de la figure 11 en position ouverte ainsi qu'en position de démoulage.

L'invention concerne un ballon intra-gastrique 1 pour le traitement de l'obésité, destiné à être implanté dans l'estomac d'un patient pour réduire le volume de l'estomac. Un tel ballon 1 comprend généralement au moins une enveloppe souple 2 délimitant un volume interne prédéterminé, ladite enveloppe souple 2 étant réalisée en un matériau élastomère, par exemple à base de silicone.

L'enveloppe souple 2 forme ainsi une poche présentant un caractère expansible, lui permettant d'occuper d'une part une configuration repliée ou lâche (non représentée), dans laquelle elle occupe un volume restreint favorisant son implantation, et d'autre part, moyennant le recours à un fluide de gonflage, une configuration expansée d'un volume prédéterminé (représentée aux figures 1 et 10), par exemple de l'ordre de 600 mL, correspondant à son volume d'utilisation dans l'estomac.

L'enveloppe souple 2 du ballon intra-gastrique 1 conforme à l'invention présente un caractère monobloc.

Dans ce qui suit, on fera référence à une enveloppe sphérique, étant entendu que l'invention ne se limite pas à cette seule forme et pourra concerner tout type de forme et par exemple les formes ellipsoïdes ou ovoïdes, sans pour autant sortir du cadre de l'invention. L'enveloppe 2 du ballon intra-gastrique 1 peut se présenter sous la forme d'une membrane homogène de forme sensiblement lisse ou régulière, ou encore sous la forme d'une membrane présentant des bosses ou lobes, tel que cela est illustré aux figures 1 et 10.

Selon une caractéristique essentielle de l'invention, la tolérance dimensionnelle T de l'épaisseur nominale $e_{nom}$ de l'enveloppe 2 du ballon intra-gastrique 1 conforme à l'invention est comprise entre 1 % et 20%, c'est-à-dire que l'épaisseur réelle e de l'enveloppe peut varier entre $e_{nom}(1 - T)$ et $e_{nom}(1 + T)$.

Par exemple, si la tolérance dimensionnelle T est de 10%, soit 0,1, l'épaisseur réelle e pourra varier, pour l'enveloppe considérée, entre $e_{nom}(1 - 0,1)$ et $e_{nom}(1 + 0,1)$, c'est-à-dire entre $0,9\ e_{nom}$ et $1,1\ e_{nom}$.

Afin de vérifier si une enveloppe donnée répond bien au critère de tolérance dimensionnelle cité plus haut, on pourra utiliser toute méthode métrologique usuellement mise en oeuvre dans le domaine industriel.

A titre purement illustratif, et de façon absolument pas limitative, une méthode mettant en oeuvre les étapes suivantes pourrait être adoptée :

- calcul de l'écart E selon la formule suivante :

$$E = 100\ (e_{max} - e_{nom})\ /\ e_{nom} \quad \text{si } |e_{max} - e_{nom}| \geq |e_{min} - e_{nom}|$$

ou

$$E = 100 \, (e_{nom} - e_{min}) / e_{nom} \qquad si \, |e_{max} - e_{nom}| \leq |e_{min} - e_{nom}|$$

où $e_{nom}$ pourrait être assimilée à la moyenne arithmétique des épaisseurs d'enveloppe relevées en un nombre N significatif de points de mesure répartis sur l'enveloppe, $e_{min}$ étant l'épaisseur minimum mesurée sur les N points tandis que $e_{max}$ est l'épaisseur maximum mesurée sur les N points.

- comparaison de E avec T :

    si $E \leq T$, il s'ensuit que le ballon objet de la vérification est conforme à l'objet de l'invention.

La mesure d'épaisseur pourra être effectuée par exemple à l'aide d'un comparateur d'épaisseur mécanique, et par exemple avec le comparateur mécanique Mitutoya NO7304. A titre d'exemple, dans le cas d'un ballon 1 présentant une forme sensiblement sphérique ou s'inscrivant sensiblement dans une sphère, le nombre de points de mesure N pourra être égal à seize, les points étant répartis de la façon suivante :

- on divise fictivement l'enveloppe 2 sensiblement sphérique à l'aide de quatre méridiens régulièrement espacés angulairement,

- on effectue des mesures en quatre points de chaque méridien, par exemple en deux points proches respectivement de chaque pôle de l'enveloppe sphérique, ainsi qu'en deux points proches de l'équateur de l'enveloppe sensiblement sphérique, et par exemple répartis de part et d'autre dudit équateur.

L'ensemble des N points de mesure doit bien sûr impérativement être choisi de façon à ce que tous les points correspondent à une même catégorie de matière. Ainsi, dans le cas où le ballon considéré est un ballon à lobes ou à facettes, tel que ceux illustrés aux figures 1 et 10, les N points devront soit être choisis de façon à être tous positionnés dans le coeur 33 de chaque facette, ou soit être choisis de façon à être tous positionnés sur les bords 32A formant les côtés de chaque facette. En effet, sur un tel ballon, les bords formant les côtés 32A de chaque facette sont généralement plus épais que le coeur 33 de chaque facette, de façon à former une surface de ballon bosselée lors du gonflement du ballon. De même, on veillera à ce que de façon générale, aucun point de mesure ne soit positionné sur une singularité du ballon, que cette singularité soit constituée par un plan de joint, un renfort (par exemple au niveau de la valve) ou tout autre élément.

Avantageusement, la tolérance dimensionnelle de l'épaisseur de l'enveloppe du ballon est comprise dans une plage allant de 10% à 16%. A titre d'exemple d'un ballon intra-gastrique conforme à l'invention, on peut citer le cas d'un ballon comprenant une enveloppe en silicone d'épaisseur nominale sensiblement égale à 0,5 mm avec une tolérance comprise entre 10% et 16%. Cela signifie que l'épaisseur nominale de la poche est de 0,5 mm, tandis que l'épaisseur réelle peut varier de $0,5 \pm 0,08$ mm (lorsque la tolérance T est égale à 16%) à $0,5 \pm 0,05$ mm (lorsque la tolérance T est égale à 10%).

Dans ce qui précède, on a envisagé le cas d'une enveloppe 2 de ballon réalisée en silicone, ou à base de silicone. Il est cependant tout à fait envisageable que cette enveloppe soit réalisée en tout autre matériau élastomère, sans sortir du cadre de l'invention.

L'invention concerne également un procédé de fabrication d'un ballon intra-gastrique 1 pour le traitement de l'obésité, ledit ballon étant destiné à être implanté dans l'estomac d'un patient pour réduire le volume de l'estomac.

Selon une caractéristique essentielle de l'invention, le procédé de fabrication comprend une étape d'injection dans laquelle on injecte un matériau élastomère, du genre silicone, dans un moule pour obtenir une poche souple destinée à former l'enveloppe 2 du ballon 1.

Avantageusement, le moule comprend :

- une empreinte supérieure 40 comprenant une conformation concave 40A définissant en creux une portion de la surface de la poche souple que l'on cherche à obtenir,

- une empreinte inférieure 41, comprenant une conformation concave 41 B qui définit en creux une surface complémentaire de celle de l'empreinte supérieure 40, de telle sorte que lorsqu'on plaque l'une contre l'autre l'empreinte supérieure 40 et l'empreinte inférieure 41 complémentaire, on obtient un volume interne fermé, sensiblement étanche, délimité par une surface 40A, 41 A dont la forme correspond à celle de la poche souple que l'on souhaite obtenir. Les empreintes supérieure 40 et inférieure 41 pourront ainsi présenter des conformations 40A, 41 A de forme générale hémisphérique ou hémiellipsoïdale par exemple. D'autres formes pourront bien entendu être envisagées, sans pour autant sortir du cadre de l'invention.

Le moule comprend également un noyau 42 formé par un corps convexe dont la surface extérieure est complémentaire de celle du volume interne défini par les empreintes supérieure 40 et inférieure 41, à un changement d'échelle près. La surface externe du noyau 42 est ainsi une homothétie réduite de la surface du volume interne défini par les empreintes supérieure 40 et inférieure 41. Le noyau 42 est destiné à être positionné au sein du volume interne, à équidistance des parois définissant le volume interne. On obtient ainsi l'agencement décrit à la figure 11, où les empreintes supérieure 40 et inférieure 41 englobent le noyau 42, de façon à définir un interstice ou entrefer 43, qui est un espace libre délimité d'une part par la surface externe 42A du noyau 42, et d'autre part par la surface interne 40A, 41 A du volume interne défini par l'empreinte supérieure 40 associée à l'empreinte inférieure complémentaire 41. Dans le cas, correspondant à celui représenté aux figures 11 et 12, où l'on souhaite obtenir une poche de forme sensiblement sphérique et monobloc, le procédé d'injection comprend ainsi, antérieurement à l'étape d'injection, une étape de préparation de moule dans laquelle on plaque l'une contre l'autre une empreinte supérieure 40 de forme générale hémisphérique et une empreinte inférieure 41 de forme générale hémisphérique complémentaire, de façon à obtenir un volume interne sensiblement en forme de sphère, en ayant au préalable positionné entre les deux empreintes 40, 41, de façon concentrique audit volume interne, un noyau sphérique 42 dont le diamètre est inférieur à celui dudit volume interne défini par les empreintes supérieure 40 et inférieure 41. Cette étape de préparation de moule est suivie d'une étape d'injection, dans laquelle on injecte un matériau élastomère, qui peut être par exemple du silicone gomme ou du silicone liquide, dans l'espace interstitiel 43 compris entre le noyau 42 et les empreintes supérieure 40 et inférieure 41, de façon à obtenir une poche de forme générale sphérique destinée à former l'enveloppe 2 du ballon intra-gastrique.

Avantageusement, l'empreinte supérieure 40 est solidarisée à une semelle supérieure 44, de façon à ce que l'espace intérieur 40B défini par la conformation concave 40A de l'empreinte 40, soit en communication fluidique avec la semelle supérieure 44, qui elle-même porte les moyens d'injection de matériau élastomère, lesquels moyens d'injection sont eux-mêmes en communication avec la presse d'injection (non représentée).

Les moyens d'injection comprennent de façon préférentielle trois buses d'injection réparties angulairement de façon régulière (espacées entre elles de 120°) autour ou au niveau du sommet 45 du volume interne défini par les empreintes supérieure 40 et inférieure 41. Le sommet 45 correspond ainsi sensiblement au point de la conformation concave 40A de l'empreinte supérieure 40 le plus proche de la semelle supérieure 44.

De façon préférentielle, les buses d'injection ont toutes un débit identique.

De façon avantageuse, le procédé met également en oeuvre une semelle inférieure 46 sur laquelle est fixé un axe de centrage 47 du noyau 42. Le noyau 42, qui se présente par exemple, tel que cela est représenté aux figures 11 et 12, sous la forme d'une sphère pleine, comprend un alésage 48 dont la forme est complémentaire de celle de l'axe de centrage 47, de façon à pouvoir emmancher de façon ajustée le noyau 42 sur l'axe 47, au travers de l'empreinte inférieure 41 qui comporte à cet effet une lumière de passage 41 B pour l'axe de centrage 47.

L'alésage 48 et de l'axe de centrage 47 sont conçus de façon à limiter tout risque de grippage entre le noyau 42 et l'axe de centrage 47. A cette fin, les aciers constituant le noyau 42 et l'axe de centrage 47 doivent préférentiellement présenter des duretés différentes, par exemple 49 ou 50 HRc pour l'axe de centrage 47, et 35 HRc pour le noyau 42 (dureté Rockwell). On envisage également de pourvoir l'axe de centrage 47 d'une embase 47A de forme générale sensiblement tronconique, ainsi que d'une extrémité d'engagement opposée 47B de forme générale également sensiblement tronconique. L'alésage 48 pratiqué dans le noyau 42 comprend, à chacune de ses extrémités, un évidement inférieur 48B, dont la forme est complémentaire de celle de l'embase 47A, ainsi qu'un évidement supérieur 48A, dont la forme est complémentaire de celle de l'extrémité d'engagement 47B, lesdits axe de centrage 47 et alésage 48 étant agencés de façon à favoriser un appui annulaire entre l'embase 47A de l'axe de centrage 47 et le noyau 42, ledit appui annulaire contribuant à la maîtrise du positionnement du noyau 42 par rapport à l'axe de centrage 47.

Afin de procéder à l'opération de démoulage, on met en oeuvre un axe de démoulage 49 dont l'une des extrémités est solidaire de la semelle inférieure 46, tandis que l'autre extrémité, ou l'extrémité d'attaque 49A, est destinée à engager l'évidement inférieur 48B du noyau 42, et présente à cet effet une conformation autorisant la mise en butée de l'extrémité inférieure 48B de l'alésage 48. Il est ainsi possible d'extraire le noyau 42 de l'empreinte inférieure 41 de la façon suivante :

- les empreintes supérieure 40 et inférieure 41 sont désolidarisées,

- puis l'empreinte inférieure 41 est soumise à un mouvement de translation verticale dans la direction selon laquelle s'étend l'axe de centrage 47, de façon à entraîner le noyau 42 hors de l'axe de centrage 47,

- puis on enfile le sous-ensemble formé par l'empreinte inférieure 41 supportant le noyau 42 sur l'axe de démoulage 49, de façon à extraire le noyau 42 hors de la conformation concave 41 A de l'empreinte inférieure 41.

On procède en dernier lieu au démoulage proprement dit de la poche de silicone épousant la surface du noyau 42. Afin de faciliter cette opération, le noyau 42 sera recouvert d'une couche uniforme de téflon® de quelques microns d'épaisseur.

Ainsi, le procédé selon l'invention permet de réaliser de façon rapide (le temps de cycle peut être de l'ordre de 5 minutes), à l'aide d'une seule machine et selon un nombre limité d'opérations, un ballon intra-gastrique monobloc présentant une grande régularité d'épaisseur de paroi. A titre d'exemple, on peut ainsi réaliser une poche en silicone d'épaisseur nominale égale à 0,5 mm avec une tolérance inférieure à $\pm$ 0,08 mm et pouvant atteindre $\pm$ 0,05 mm.

De nombreuses formes de ballon sont réalisables grâce au procédé conforme à l'invention, et notamment des formes de ballon alvéolées ou à facettes.

POSSIBILITE D'APPLICATION INDUSTRIELLE

L'invention trouve son application industrielle dans la réalisation et l'utilisation de ballons intra-gastriques de traitement de l'obésité.

**Revendications**

1. Ballon intra-gastrique (1) pour le traitement de l'obésité, destiné à être implanté dans l'estomac d'un patient pour réduire le volume de l'estomac, ledit ballon (1) comprenant une enveloppe souple (2) délimitant un volume interne prédéterminé, ladite enveloppe souple (2) étant réalisée en un matériau élastomère, **caractérisé en ce que** la tolérance dimensionnelle (T) de l'épaisseur nominale de l'enveloppe est comprise entre 1% et 20%.

2. Ballon intra-gastrique (1) selon la revendication 1 **caractérisé en ce que** la tolérance est comprise dans une plage allant de 10% à 16%.

3. Ballon intra-gastrique selon l'une des revendications 1 ou 2 **caractérisé en ce que** l'épaisseur nominale de l'enveloppe (2) est sensiblement égale à 0,5 mm avec une tolérance (T) comprise entre 10% et 16%.

4. Ballon intra-gastrique (1) selon l'une des revendications 1 à 3 **caractérisé en ce que** l'enveloppe (2) est en silicone, ou à base de silicone.

5. Procédé de fabrication d'un ballon intra-gastrique (1) selon la revendication 1
**caractérisé en ce qu'**il comprend une étape d'injection dans laquelle on injecte un matériau élastomère dans un moule pour obtenir une poche souple destinée à former l'enveloppe (2) du ballon (1).

6. Procédé selon la revendication 5 **caractérisé en ce qu'**il comprend, antérieurement à l'étape d'injection, une étape de préparation de moule dans laquelle on plaque l'une contre l'autre une empreinte supérieure (40) de forme générale hémisphérique et une empreinte inférieure (41) de forme générale hémisphérique complémentaire, de façon à obtenir un volume interne sensiblement en forme de sphère, en ayant au préalable positionnée entre les deux empreintes (40, 41), de façon concentrique, un noyau sphérique (42) dont le diamètre est inférieur à celui dudit volume interne, l'étape d'injection comprenant une étape d'injection du matériau élastomère dans l'espace (43) compris entre le noyau (42) et les empreintes (40, 41), de façon à obtenir une poche de forme générale sphérique destinée à former l'enveloppe (2) du ballon.

**Claims**

1. Intragastric balloon (1) for the treatment of obesity, intended to be implanted in a patient's stomach in order to reduce the volume of the stomach, the said balloon (1) comprising a flexible casing (2) delimiting a predetermined internal volume, the said flexible casing (2) being made of an elastomeric material, **characterized in that** the dimensional tolerance (T) of the nominal thickness of the casing lies between 1% and 20%.

2. Intragastric balloon (1) according to Claim 1, **characterized in that** the tolerance lies in an interval ranging from 10% to 16%.

3. Intragastric balloon (1) according to one of Claims 1 and 2, **characterized in that** the nominal thickness of the casing (2) is substantially equal to 0.5 mm with a tolerance (T) lying between 10% and 16%.

**4.** Intragastric balloon (1) according to one of Claims 1 to 3, **characterized in that** the casing (2) is made of silicone or based on silicone.

**5.** Method for manufacturing an intragastric balloon (1) according to Claim 1, **characterized in that** it comprises an injection step in which an elastomeric material is injected into a mould in order to obtain a flexible shell intended to form the casing (2) of the balloon (1).

**6.** Method according to Claim 5, **characterized in that** it comprises, prior to the injection step, a mould preparation step in which an upper impression (40) of hemispherical general shape and a lower impression (41) of complementary hemispherical general shape are pressed against one another so as to obtain an internal volume substantially in the form of a sphere, while having previously positioned a spherical core (42) concentrically between the two impressions (40, 41), the diameter of which core is less than the said internal volume, the injection step comprising a step of injecting the elastomeric material into the space (43) contained between the core (42) and the impressions (40, 41), so as to obtain a shell of spherical general shape intended to form the casing (2) of the balloon.

**Patentansprüche**

**1.** Intragastrischer Ballon (1) zur Behandlung von Fettleibigkeit, der in den Magen eines Patienten implantiert werden soll, um das Volumen des Magens zu verringern, wobei der Ballon (1) eine flexible Hülle (2) umfasst, die ein zuvor festgelegtes inneres Volumen umschließt, wobei die flexible Hülle (2) aus einem Elastomermaterial hergestellt wird, **dadurch gekennzeichnet, dass** die Maßtoleranz (T) der nominellen Dicke der Hülle zwischen 1% und 20% beträgt.

**2.** Intragastrischer Ballon (1) nach Anspruch 1, **dadurch gekennzeichnet, dass** die Toleranz in einem Bereich von 10% bis 16% liegt.

**3.** Intragastrischer Ballon nach einem der Ansprüche 1 oder 2, **dadurch gekennzeichnet, dass** die nominelle Dicke der Hülle (2) im Wesentlichen gleich 0,5 mm ist, wobei die Toleranz (T) zwischen 10% und 16% beträgt.

**4.** Intragastrischer Ballon (1) nach einem der Ansprüche 1 bis 3, **dadurch gekennzeichnet, dass** die Hülle (2) aus Silikon oder auf Silikonbasis ist.

**5.** Verfahren zur Herstellung eines intragastrischen Ballons (1) nach Anspruch 1, **dadurch gekennzeichnet, dass** es einen Einspritzschritt umfasst, bei dem man ein Elastomermaterial in eine Form einspritzt, um eine flexible Tasche zu erhalten, die die Hülle (2) des Ballons (1) bilden soll.

**6.** Verfahren nach Anspruch 5, **dadurch gekennzeichnet, dass** es vor dem Einspritzschritt einen Schritt umfasst, bei dem man die Form vorbereitet, in die man eine im Großen und Ganzen halbkugelförmige obere Vertiefung (40) und eine im Großen und Ganzen halbkugelförmige, komplementäre untere Vertiefung (41) derart gegeneinander presst, dass man ein im Wesentlichen kugelförmiges inneres Volumen erhält, wobei man zuvor zwischen die beiden Vertiefungen (40, 41) einen kugelförmigen Kern (42) konzentrisch eingebracht hat, dessen Durchmesser kleiner als derjenige des inneren Volumens ist, wobei der Einspritzschritt einen Schritt umfasst, bei dem man das Elastomermaterial in den Zwischenraum (43) zwischen dem Kern (42) und den Vertiefungen (40, 41) einspritzt, so dass eine im Großen und Ganzen kugelförmige Tasche erhalten wird, die die Hülle (2) des Ballons bilden soll.

FIG.2

FIG.1

FIG.3

FIG.4          FIG.5          FIG.6

EP 1 458 316 B1

FIG.7

FIG.8

FIG.9

FIG.10

FIG.11

FIG.12

**EP 1 458 316 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5084061 A
- US 4694827 A